(19) Europäisches Patentamt
European Patent Office
Office européen des brevets

(11) Numéro de publication : **0 446 086 B1**

# (12) FASCICULE DE BREVET EUROPEEN

(45) Date de publication du fascicule du brevet : **08.12.93 Bulletin 93/49**

(51) Int. Cl.$^5$ : **C07C 15/16,** C07C 2/86, H01B 3/22

(21) Numéro de dépôt : **91400361.1**

(22) Date de dépôt : **13.02.91**

(54) **Composition à base de dérivés méthyles et benzyles du diphénylméthane, son application comme diélectrique.**

(30) Priorité : **27.02.90 FR 9002421**

(43) Date de publication de la demande : **11.09.91 Bulletin 91/37**

(45) Mention de la délivrance du brevet : **08.12.93 Bulletin 93/49**

(84) Etats contractants désignés : **AT BE CH DE DK ES FR GB GR IT LI LU NL SE**

(56) Documents cités :
**EP-A- 0 136 230**
**EP-A- 0 299 867**

(73) Titulaire : **ELF ATOCHEM S.A.**
**4 & 8, Cours Michelet La Défense 10**
**F-92800 Puteaux (FR)**

(72) Inventeur : **Berger, Noelle**
**Frênes 4, Charrière Blanche**
**F-69130 Ecully (FR)**
Inventeur : **Commandeur, Raymond**
**Le Rocher, Avenue de Vénaria**
**F-38220 Vizille (FR)**
Inventeur : **Jay, Pierre**
**89, Rocade des Monts d'Or**
**F-69370 Saint-Didier au Mont d'Or (FR)**

EP 0 446 086 B1

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

## Description

La présente invention concerne une composition à base de dérivés méthylés et benzylés du diphénylméthane et son application comme diélectrique.

Le brevet européen EP 136 230 décrit des diélectriques qui restent liquides à basse température sans présenter une viscosité élevée. On a maintenant trouvé une nouvelle composition diélectrique qui reste liquide à des températures encore plus basses. Un autre but de l'invention est de fournir une composition diélectrique utilisable dans les transformateurs de tension, c'est-à-dire qu'elle doit avoir une tension de claquage élevée.

La présente invention est donc une composition comprenant du benzyltoluène, du benzylxylène, du (méthylbenzyl)toluène et du (méthylbenzyl)xylène.

On désigne par benzyltoluène, un isomère ou un mélange d'isomères de formule :

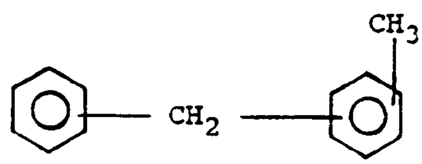

On désigne par benzylxylène un isomère ou un mélange d'isomères de formule :

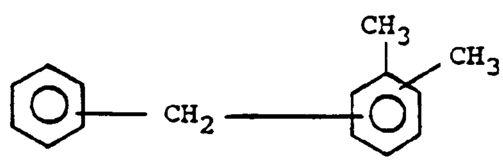

On désigne par (méthylbenzyl)toluène un isomère ou un mélange d'isomères de formule :

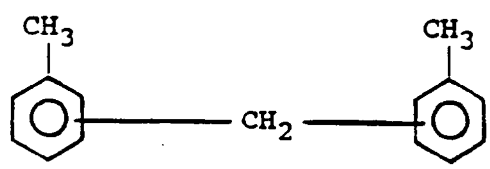

On désigne par (méthylbenzyl) xylène un isomère ou un mélange d'isomères de formule :

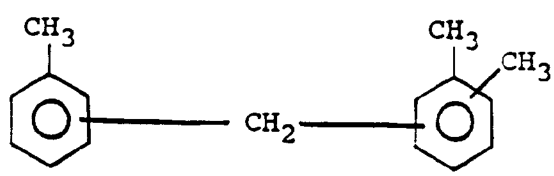

La composition selon l'invention comprend aussi de préférence un isomère ou un mélange d'isomères de formule A :

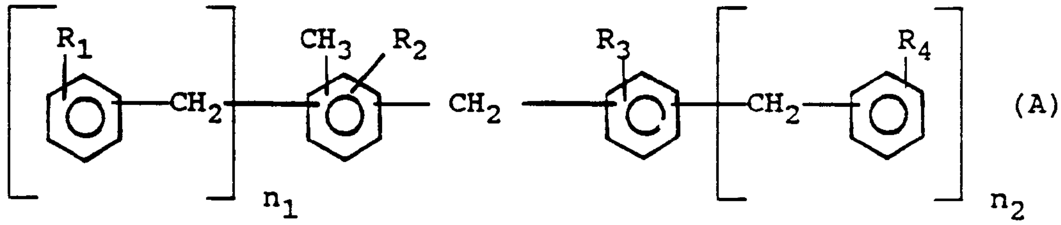

(A)

dans laquelle $R_1$, $R_2$, $R_3$ et $R_4$ sont identiques ou différents et désignent de l'hydrogene ou un méthyle, $n_1$ et

$n_2$ valent 0, 1 ou 2 avec la condition $n_1 + n_2$ est supérieur ou égal à 1 et inférieur ou égal à 3.

A titre d'exemple quand $R_1$ à $R_4$ sont des hydrogene et $n_1 + n_2 = 1$, le produit A désigne le dibenzyltoluène. Mais le produit A peut aussi être un mélange d'isomères contenant des produits tels que $n_1 + n_2 = 1$, $n_1 + n_2 = 2$ et $n_1 + n_2 = 3$.

On ne sortirait pas du cadre de l'invention si la composition de l'invention contenait aussi des triphénylméthane pouvant être substitués par des méthyls, des benzyls et des méthylbenzyls. A titre d'exemple de ces triphénylméthanes substitués, on peut citer le ditolylphénylméthane, le dixylylphénylméthane ou encore le xylyltolylphénylméthane.

La composition selon l'invention est utile comme diélectrique. On peut par exemple l'utiliser dans les condensateurs, les transformateurs, ou dans les câbles électriques.

Selon les applications diélectriques, il est avantageux de purifier la composition de l'invention à l'aide de terres décolorantes, zéolites, puis d'ajouter des anti-oxydants ou des accepteurs d'acides tels que des époxydes. Tous ces conditionnements sont connus de l'homme de métier, on peut se reporter par exemple aux brevets européens EP 8251 et EP 136 230 dont le contenu est incorporé dans la présente demande.

On ne sortirait pas du cadre de l'invention si on utilisait la composition selon l'invention en mélange avec d'autres diélectriques, par exemple des tétrachlorobenzyltoluènes et des chlorobenzènes ou chlorotoluènes décrits dans le brevet européen EP 8251 ou des huiles minérales habituellement utilisées dans les transformateurs.

On utilise avantageusement des compositions comprenant au moins 60 parties de produits à 2 noyaux benzéniques (c'est-à-dire benzyltoluène, benzylxylène, (méthylbenzyl)toluène et (méthylbenzyl)xylène) pour 40 parties d'isomères de formule A.

Selon une autre forme avantageuse de l'invention, parmi les 4 produits à 2 noyaux benzéniques le benzyltoluène représente au moins 30 % en poids de l'ensemble de ces 4 produits, avantageusement 50% et de préférence 70 %.

Des compositions particulièrement préférées sont telles que la quantité des 4 produits à 2 noyaux est de 65 à 90 parties pour respectivement 35 à 10 parties d'isomères de formule A.

Les différents produits ou isomères de la composition selon l'invention, c'est-à-dire des dérivés méthylés et benzylés du diphénylméthane, peuvent être fabriqués selon différents procédés, puis mélangés. Cependant, l'un des buts de l'invention est aussi de proposer un procédé particulièrement simple pour fabriquer la composition de l'invention.

En effet, dans les brevets européens EP 136 230 et EP 299 867, au nom de la demanderesse, on cite une préparation du benzyltoluène et du (méthylbenzyl)xylène. Dans la demande de brevet européen EP 282 083 on décrit la production d'un mélange de benzyltoluène et de (méthylbenzyl)toluène, par des réactions d'alkylation et transalkylation à haute température qui doivent être suivies de purifications complexes. Le procédé selon l'invention est caractérisé en ce que dans une première étape on effectue une chloration radicalaire partielle d'un mélange de toluène et de xylène, puis dans une deuxième étape on met en contact le mélange résultant de cette première étape avec un catalyseur de FRIEDEL et CRAFTS.

La chloration radicalaire est connue en soi, on peut utiliser un initiation chimique ou par ultra-violets.

La chloration radicalaire du mélange de toluène et de xylène, est habituellement réalisée à une température comprise entre 50 et 110°C et mieux entre 70 et 100°C. Elle est de préférence menée de telle sorte qu'on ne transforme que 10 à 30 %, exprimé en pourcentage molaire, du mélange engagé en dérivé chloré correspondant. En tant que générateur de radicaux libres, on peut employer soit une initiation photochimique, soit un initiateur chimique ; parmi les initiateurs chimiques, on peut citer les composés azo comme l'azodiisobutyronitrile ou encore l'azodivaléronitrile, ou des péroxydes tels que le péroxyde de lauroyle. La quantité d'initiateur chimique mise en oeuvre est généralement comprise entre 0,05 et 3 % en poids par rapport au mélange engagé, et de préférence entre 0,1 et 1,5 %.

La chloration doit être partielle, c'est-à-dire qu'à la fin de cette première étape, il doit rester dans le mélange réactionnel du xylène et du toluène. La proportion initiale de toluène et de xylène, c'est-à-dire avant la chloration, peut être quelconque, elle est d'autant plus élevée en toluène qu'on souhaite plus de benzyltoluène. La deuxième étape consiste à mettre en contact le mélange précédent avec un catalyseur de FRIEDEL et CRAFTS. Ces catalyseurs sont connus en eux-mêmes.

On peut utiliser par exemple comme catalyseur un halogénure minéral, ou encore un acide minéral. Cette réaction a lieu en pratique à une température comprise entre 30 et 110°C, et de préférence entre 50 et 100°C. Parmi les halogénures minéraux, on peut utiliser le chlorure ferrique, le trichlorure d'antimoine, le tétrachlorure de titane ou encore le chlorure d'aluminium à des teneurs pondérales par rapport au milieu réactionnel compris habituellement entre 50 ppm et 1 % et de préférence entre 100 ppm et 0,5 %. Les acides minéraux peuvent également être utilisés : l'acide sulfurique par exemple à une concentration pondérale comprise entre 70 et 95 %. Il est aussi possible d'employer les zéolites ou encore certains oxydes minéraux.

Avantageusement, on verse le catalyseur dans le mélange précédent.

Avantageusement, à la fin de cette deuxième étape, qui est en fait une réaction de condensation, il est recommandé après distillation du toluène et du xylène en excès, de procéder à l'élimination du catalyseur par toute technique connue telle que : lavage à l'eau, neutralisation, séchage.

Il suffit ensuite d'une simple distillation pour récupérer la composition de l'invention. On peut aussi par distillation séparer ces 4 produits à deux noyaux des produits A et éventuellement des produits plus lourds. Le mélange réactionnel qu'on vient d'obtenir après la condensation et qui est constitué essentiellement de dérivés méthylés et benzylés du diphénylméthane et d'un excès éventuel de xylène et toluène peut contenir des produits chlorés organiques tels que :

- des xylènes chlorés de formule :

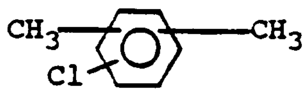

- des chlorotoluènes de formule :

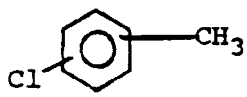

- du (méthylchlorobenzyl)xylène :

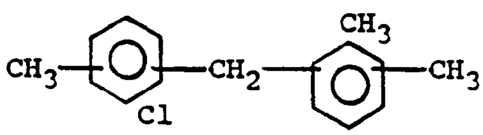

et d'une façon générale des dérivés méthylés et benzylés du diphénylméthane portant un ou plusieurs atomes de chlore sur le noyau benzénique. Ces produits ont été formés à partir du chlore et du xylène et toluène ou éventuellement par le catalyseur de FRIEDEL et CRAFTS quand c'est un chlorure. Pour certaines applications, il est souhaitable que les produits de l'invention contiennent très peu ou pas du tout de chlore. Selon une forme particulière de l'invention, on traite les dérivés méthylés et benzylés du diphénylméthane pour éliminer les produits chlorés organiques. Il est recommandé d'effectuer le traitement après l'élimination du xylène, du toluène en excès et du catalyseur.

Après la réaction de condensation on élimine donc le xylène et le toluène en excès, puis le catalyseur et sur ce mélange brut de dérivés méthylés et benzylés du diphénylméthane on effectue une élimination du chlore organique.

On peut utiliser tout procédé de destruction des produits chlorés organiques, par exemple le procédé décrit dans EP 306 398 utilisant un alcoolate de métal alcalin, le procédé décrit dans EP 250 748 utilisant un alcoolate lourd, le contenu de ces demandes étant incorporé dans la présente invention. On préfère utiliser le procédé de déchloration décrit dans EP 306 398.

Selon le procédé préféré, on met en contact le mélange brut avec un alcoolate et on porte l'ensemble sous agitation à une température comprise entre 220 et 320°C. L'alcoolate est de préférence un alcoolate de sodium, par exemple du méthylate de sodium.

Après le traitement de déchloration, il suffit d'une simple distillation pour récupérer les dérivés méthylés et benzylés du diphénylméthane à faible teneur en chlore. On obtient en pied une fraction lourde contenant les restes de l'agent déchlorant, un chlorure alcalin (NaCl) et des oligomères lourds.

On ne sortirait pas du cadre de l'invention si on recyclait, en partie ou en totalité, cette fraction lourde obtenue en pied et qu'on l'utilisait seule ou en mélange avec le produit mis en oeuvre pour détruire les produits chlorés organiques.

On ne sortirait pas du cadre de l'invention si après la condensation et après élimination du xylène et du toluène, on recyclait à l'étape de condensation, en tout ou partie, les produits à deux noyaux (benzyltoluène, benzylxylène, (méthylbenzyl)xylène, (méthylbenzyl)toluène). L'avantage de ce recyclage est d'augmenter la proportion de produits de formule A. Le xylène et le toluène éliminés peuvent être réutilisés en amont dans le procédé. On pourrait aussi prendre tout ou partie des produits à deux noyaux après la déchloration et les recycler à l'étape de condensation.

On ne sortirait pas du cadre de l'invention si on effectuait un mélange de chlorure de benzyle, de chlorure de méthylbenzyle ($CH_3C_6H_4CH_2Cl$) de xylène et de toluène, puis qu'on effectuait sur ce mélange la condensation de la deuxième étape par addition de catalyseur de FRIEDEL et CRAFTS.

La demanderesse a découvert aussi que si on effectue le procédé de l'invention avec du chlorure ferrique comme catalyseur de FRIEDEL et CRAFTS, alors il n'est pas nécessaire d'éliminer le catalyseur, c'est-à-dire qu'on peut distiller le mélange obtenu après la réaction de condensation pour obtenir, d'abord le xylène, le toluène, puis les 4 produits à deux noyaux et les produits A ; tous ces produits étant aptes à l'usage diélectrique. Cette propriété n'est pas vraie pour le chlorure d'aluminium, si on n'élimine pas le chlorure d'aluminium après la condensation, très souvent on ne peut même pas distiller le mélange. Si on arrive à récupérer des produits, alors ils sont inaptes à l'usage diélectrique. La demanderesse a aussi découvert que le fait d'utiliser le chlorure ferrique comme catalyseur de condensation permet non seulement d'éviter son traitement d'élimination, mais qu'on peut procéder dès la fin de la condensation à la destruction éventuelle des produits chlorés organiques comme on l'a décrit précédemment. Il est quand même recommandé d'éliminer le xylène et le toluène en excès dès la fin de réaction de condensation.

Après le traitement de déchloration, il suffit d'une simple distillation pour récupérer les 4 produits à deux noyaux et les produits A à faible teneur en chlore. On obtient en pied une fraction lourde contenant les restes de l'agent déchlorant, du chlorure alcalin, des sels de fer et des oligomères lourds. Comme précédemment cette fraction lourde peut être recyclée et servir d'appoint à l'agent déchlorant.

## EXEMPLE 1

Dans un réacteur muni d'une agitation, d'un condenseur, d'un tube d'alimentation de chlore, et d'une lampe PHILIPS TLADK de 30 Watt, on place :

40 moles de toluène ---> 3680 g
3,25 moles d'orthoxylène ---> 344,5 g

et on introduit 10,8 moles de chlore gazeux en 2 h 40 à la température de 100°C.

Après arrêt de l'initiation photochimique et dégazage à l'azote, le milieu réactionnel est placé dans une ampoule de coulage et il est introduit en 2 h dans un réacteur muni d'une agitation et contenant :

42,36 moles de toluène ---> 3897 g
3,44 moles d'orthoxylène ---> 364 g et
2,1 g de $FeCl_3$

La température de la réaction est de 100°C. On ajoute encore 1,3 g de $FeCl_3$ et la température est maintenue encore 2 heures avec dégazage par l'azote. L'excès de toluène et d'orthoxylène est éliminé par distillation sous vide de 15 mm de mercure avec une colonne de quelques plateaux. Le résidu est ensuite traité pendant 5 h à 290°C avec 2,2 % de méthylate de sodium sous couverture d'azote dans un réacteur agité. Le mélange issu de ce traitement est soumis à une distillation avec quelques plateaux sous 0,5 mm de mercure. Nous obtenons :

**1°)** Une fraction d'un liquide incolore distillant à la température de 110-125°C ayant la composition suivante :

| | |
|---|---|
| benzyltoluène | : 71,6 % |
| (méthylbenzyl)toluène | : 13,2 % |
| benzylxylène | : 12,5 % |
| (méthylbenzyl)xylène | : 2,7 % |

**2°)** Une fraction d'un liquide jaune clair huileux distillant à la température de 190-200°C comprenant un mélange de composés de type A dans lequel $n_1 + n_2$ est égal à 1. La teneur en dibenzyltoluène de cette fraction est d'environ 50 %.

La première fraction représente 1200 g, la deuxième représente 300 g. Il reste environ 60 g d'un mélange de résidus lourds.

## EXEMPLE 2

On a fait des essais de cristallisation sur :

**a)** Un liquide qu'on appelle BTXX, conforme à l'invention, et constitué de :

---> 83 parties de la 1ère fraction de l'exemple 1

---> 17 parties de la 2ème fraction de l'exemple 1 (c'est-à-dire un mélange de 14 parties de composés de type $A/n_1+n_2=1$ et 3 parties de dérivés du triphénylméthane)

**b)** Un liquide qu'on appelle BT06, non conforme à l'invention, correspondant au brevet EP 136 230 et constitué de :

---> 79 parties de benzyltoluène

---> 16 parties de dibenzyltoluène, c'est-à-dire le produit A de la présente invention dans lequel $R_1$ à $R_4$ sont des H et $n_1+n_2=1$)

---> 4 parties de ditolylphénylméthane.

Les tubes contenant le BT06 et le BTXX ont été maintenus 43 jours à -50°C. Ils ont été ensemencés avec des cristaux de benzyltoluène.

A -45°C les 2/3 du BT06 sont cristallisés alors que le BTXX est encore liquide.

A -50°C le BTXX est encore liquide.

Après agitation à -50°C le BT06 présente une quantité importante de cristaux alors que le BTXX reste liquide.

## EXEMPLE 3

Mesure des rigidités

Les mesures sont effectuées en alternatif 50 H3 température ambiante avec électrodes $\phi$ = 0, 6 mm et disque ROGOWSKI $\phi$ = 40 mm. La tension était appliquée par paliers avec une augmentation de 1000 volts toutes les 30 secondes.

| Distance interélectrode | Tension de claquage | |
|---|---|---|
| | BTXX | PXE |
| 3,2 mm | 54,6 | 40,2 |
| 10 mm | 72,0 | 63,3 |

Les valeurs sont en Kilovolts et sont des moyennes sur 5 mesures.

BTXX désigne le produit conforme à l'invention expliqué dans l'exemple 2.

PXE désigne un diélectrique non conforme à l'invention et qui est un mélange d'isomères du phényl -1-xylyl-1 éthane de formule

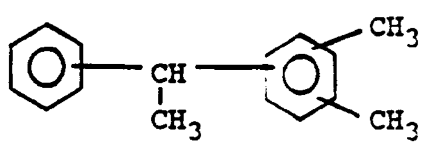

## Revendications

1. Composition comprenant du benzyltoluène, du benzylxylène, du (méthylbenzyl)toluène et du (méthylbenzyl)xylène.

2. Composition selon la revendication 1, caractérisée en ce qu'elle comprend aussi un isomère ou un mélange d'isomères de formule :

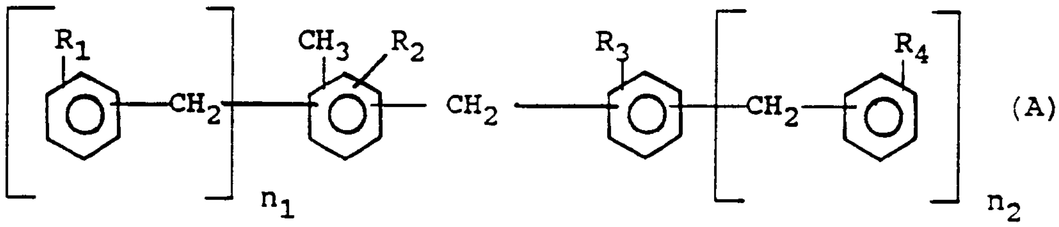

(A)

dans laquelle $R_1$, $R_2$, $R_3$ et $R_4$ sont identiques ou différents et désignent H ou $CH_3$ , $n_1$ et $n_2$ valent 0, 1 ou 2 avec la condition $n_1 + n_2$ vaut 1, 2 ou 3.

3. Composition selon la revendication 2, caractérisée en ce qu'elle comprend au moins 60 parties de produit à 2 noyaux benzéniques pour 40 parties d'isomères de formule A.

4. Composition selon la revendication 3, caractérisée en ce que, parmi les 4 produits à deux noyaux benzéniques, le benzyltoluène représente au moins 30 % en poids de l'ensemble de ces 4 produits avantageusement 50% et de préférence 70 %.

5. Application des compositions selon l'une des revendications 1 à 4 comme diélectrique, notamment pour les transformateurs, les condensateurs et les câbles électriques.

6. Procédé de synthèse de dérivés méthylés et benzylés du diphénylméthane caractérisé en ce que dans une première étape on effectue une chloration radicalaire partielle d'un mélange de toluène et de xylène, puis dans une deuxième étape on met en contact le mélange résultant de cette première étape avec un catalyseur de FRIEDEL et CRAFTS.

7. Procédé selon la revendication 6, caractérisé en ce que dans la première étape on ne transforme que 10 à 30 % molaire du mélange xylène et toluène en dérivé chloré correspondant.

8. Procédé selon la revendication 6 ou 7, caractérisé en ce qu'après la condensation on élimine les produits chlorés organiques par réaction avec du sodium ou un alcoolate de métal alcalin et de préférence du méthylate de sodium.

9. Procédé selon l'une des revendications 6 à 8, caractérisé en ce qu'on recycle en tout ou partie les produits à deux noyaux benzéniques à l'étape de condensation.

10. Procédé selon l'une des revendications 8 et 9, caractérisé en ce qu'on recycle en partie ou en totalité, à l'étape d'élimination des produits chlorés organiques, la fraction lourde qu'on obtient dans la distillation pour récupérer les dérivés méthylés et benzylés du diphénylméthane à faible teneur en chlore.

11. Procédé selon l'une des revendications 6 à 10, caractérisé en ce qu'on utilise le chlorure ferrique comme catalyseur de condensation.

12. Procédé selon la revendication 11, caractérisé en ce qu'on effectue l'élimination des produits chlorés organiques sans avoir éliminé le chlorure ferrique.

13. Procédé de synthèse de dérivés méthylés et benzylés du diphénylméthane caractérisé en ce qu'on effectue la condensation d'un mélange de chlorure de benzyle et de chlorure de méthylbenzyle sur un mélange de toluène et de xylène en présence d'un catalyseur de FRIEDEL et CRAFTS.

## Claims

1. Composition comprising benzyltoluene, benzylxylene, (methylbenzyl)toluene and (methylbenzyl)xylene.

2. Composition according to Claim 1, characterized in that it also comprises an isomer or a mixture of isomers of formula:

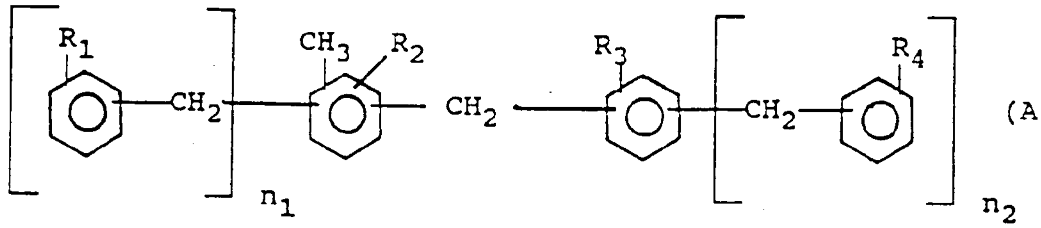

(A)

in which $R_1$, $R_2$, $R_3$ and $R_4$ are identical or different and designate H or $CH_3$, $n_1$ and $n_2$ are 0, 1 or 2 under the condition that $n_1 + n_2$ are 1, 2 or 3.

3. Composition according to Claim 2, characterized in that it comprises at least 60 parts of the product having 2 benzene rings per 40 parts of isomers of the formula A.

4. Composition according to Claim 3, characterized in that, of the 4 products having two benzene rings, benzyltoluene represents at least 30 % by weight of the total of these 4 products, advantageously 50 %, and preferably 70 %.

5. Application of the compositions according to one of Claims 1 to 4 as a dielectric, especially for transformers, capacitors and electric cables.

6. Process for the synthesis of methylated and benzylated derivatives of diphenylmethane, characterized in that in a first step a partial radical chlorination of a mixture of toluene and xylene is carried out, followed in a second step by bringing the mixture resulting from this first step into contact with a Friedel-Crafts catalyst.

7. Process according to Claim 6, characterized in that in the first step only 10 to 30 mol% of the xylene and toluene mixture are converted into the corresponding chlorinated derivative.

8. Process according to Claim 6 or 7, characterized in that after the condensation reaction, the chlorinated organic products are removed by reaction with sodium or an alkali metal alcoholate and preferably sodium methoxide.

9. Process according to one of Claims 6 to 8, characterized in that the products having two benzene rings are recycled entirely or in part to the condensation step.

10. Process according to one of Claims 8 and 9, characterized in that the heavy fraction obtained in the distillation for recovering the methylated and benzylated derivatives of diphenylmethane which have a low chlorine content is recycled in part or entirely into the step of removing the chlorinated organic products.

11. Process according to one of Claims 6 to 10, characterized in that ferric chloride is used as the condensation catalyst.

12. Process according to Claim 11, characterized in that the chlorinated organic products are removed without the ferric chloride having been removed.

13. Process for the synthesis of methylated and benzylated derivatives of diphenylmethane, characterized in that the condensation of a mixture of benzyl chloride and methylbenzyl chloride with a mixture of toluene and xylene is carried out in the presence of a Friedel-Crafts catalyst.

**Patentansprüche**

1. Zusammensetzung, enthaltend Benzyltoluol, Benzylxylol, (Methylbenzyl)toluol und (Methylbenzyl) xylol.

2. Mischung nach Anspruch 1, dadurch gekennzeichnet, daß sie auch ein Isomeres oder ein Gemisch von Isomeren der Formel

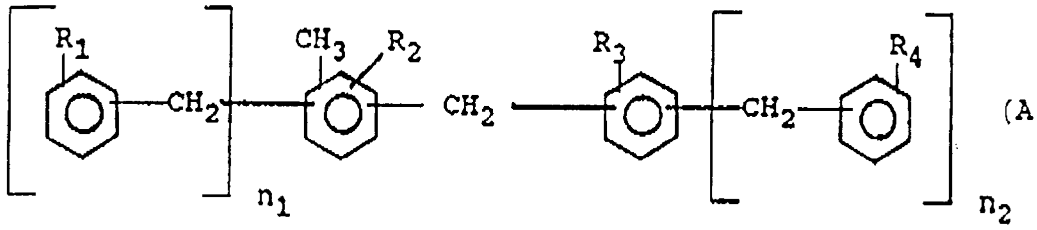

(A)

enthält,
in der $R_1$, $R_2$, $R_3$ und $R_4$ gleich oder verschieden sind und H oder $CH_3$ symbolisieren, $n_1$ und $n_2$ 0, 1 oder 2 betragen, mit der Bedingung $n_1 + n_2$ gleich 1, 2 oder 3.

3. Zusammensetzung nach Anspruch 2, dadurch gekennzeichnet, daß sie mindestens 60 Teile des zweikernigen aromatischen Produktes auf 40 Teile der Isomeren der Formel A enthält.

4. Zusammensetzung nach Anspruch 3, dadurch gekennzeichnet, daß unter den 4 zweikernigen aromatischen Produkten das Benzyltoluol mindestens 30 Gewichtsprozente, vorteilhafterweise 50 % und vorzugsweise 70 %, der Gesamtheit dieser 4 Produkte darstellt.

5. Anwendung der Mischungen nach einem der Ansprüche 1 bis 4 als Dielektrikum, insbesondere für Transformatoren, Kondensatoren und elektrische Kabel.

6. Verfahren zur Herstellung von Methyl- und Benzylderivaten des Diphenylmethans, dadurch gekennzeichnet, daß man in einer ersten Stufe eine teilweise radikalische Chlorierung eines Gemisches von Toluol und Xylol durchführt und dann in einer zweiten Stufe das in dieser ersten Stufe erhaltene Gemisch mit einem FRIEDEL-CRAFTS-Katalysator zusammenbringt.

7. Verfahren nach Anspruch 6, dadurch gekennzeichnet, daß man in der ersten Stufe nur 10 bis 30 Mol.% des Xylol-Toluolgemisches in entsprechende chlorierte Derivate umwandelt.

8. Verfahren nach Anspruch 6 oder 7, dadurch gekennzeichnet, daß man nach der Kondensation die chlorierten organischen Produkte durch Reaktion mit Natrium oder einem Alkalialkoholat und bevorzugt mit Natriummethanolat entfernt.

9. Verfahren nach einem der Ansprüche 6 bis 8, dadurch gekennzeichnet, daß man in der Kondensationsstufe die zweikernigen aromatischen Produkte vollständig oder teilweise recycliert.

10. Verfahren nach einem der Ansprüche 8 und 9, dadurch gekennzeichnet, daß man in der Stufe zur Entfernung der chlorierten organischen Produkte die bei der Destillation zur Rückgewinnung der Methyl- und Benzylderivate des Diphenylmethans mit geringem Chlorgehalt erhaltene hochsiedende Fraktion teilweise oder vollständig recycliert.

11. Verfahren nach einem der Ansprüche 6 bis 10, dadurch gekenzeichnet, daß man als Kondensationskatalysator Eisen(III)-chlorid einsetzt.

12. Verfahren nach Anspruch 11, dadurch gekennzeichnet, daß man die Entfernung der chlorierten organischen Produkte vornimmt, ohne das Eisen(III)-chlorid entfernt zu haben.

13. Verfahren zur Synthese von Methyl- und Benzylderivaten des Diphenylmethans, dadurch gekennzeichnet, daß man die Kondensation eines Gemisches aus Benzylchlorid und Methylbenzylchlorid in einer Mischung aus Toluol und Xylol in Gegenwart eines FRIEDEL-CRAFS-Katalysators durchführt.